(19) Europäisches Patentamt
European Patent Office
Office européen des brevets

(11) Veröffentlichungsnummer: **0 002 214**
**B1**

(12) **EUROPÄISCHE PATENTSCHRIFT**

(45) Veröffentlichungstag der Patentschrift: **13.05.81**

(21) Anmeldenummer: **78101394.1**

(22) Anmeldetag: **18.11.78**

(51) Int. Cl.³: **C 07 C 43/184,**
C 07 C 41/16,
C 07 C 43/192,
A 01 N 31/14 //C07C35/06,
C07C35/08, C07C49/487

# (54) Benzyläther von cyclischen 1,2-Diolen, Verfahren zu ihrer Herstellung sowie ihre Verwendung als Herbizide.

(30) Priorität: **01.12.77 DE 2753556**
**28.09.78 DE 2842188**

(43) Veröffentlichungstag der Anmeldung:
**13.06.79 Patentblatt 79/12**

(45) Bekanntmachung des Hinweises auf die Patenterteilung:
**13.05.81 Patentblatt 81/19**

(84) Benannte Vertragsstaaten:
**BE CH DE FR GB NL SE**

(56) Entgegenhaltungen:
**FR - A - 1 176 120**

(73) Patentinhaber: **BAYER AG**
**Zentralbereich Patente, Marken und Lizenzen**
**D-5090 Leverkusen 1, Bayerwerk (DE)**

(72) Erfinder: **Draber, Wilfried, Dr.**
**In den Birken 81**
**D-5600 Wuppertal 1 (DE)**
Erfinder: **Reiser, Wolf, Dr.**
**Kiebitzweg 17**
**D-5600 Wuppertal 1 (DE)**
Erfinder: **Schmidt, Thomas, Dr.**
**Wormser Strasse 23**
**D-5600 Wuppertal 1 (DE)**
Erfinder: **Eue, Ludwig, Dr.**
**Paul-Klee-Strasse 36**
**D-5090 Leverkusen 1 (DE)**
Erfinder: **Schmidt, Robert Rudolf, Dr.**
**Hahnenweg 5**
**D-5000 Köln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 002 214 B1

Benzyläther von cyclischen 1,2-Diolen, Verfahren zu ihrer Herstellung sowie ihre Verwendung als Herbizide

Die vorliegende Erfindung betrifft neue Benzyläther von cyclischen 1,2-Diolen, ein Verfahren zu ihrer Herstellung sowie ihre Verwendung als Herbizide, insbesondere als selektive Herbizide.

Es ist bereits bekannt geworden, daß Chloracetanilide, wie beispielsweise 2-Aethyl-6-methyl-N-(1'-methyl-2'-methoxyäthyl)-chloracetanilid, als Herbizide, insbesondere zur Bekämpfung von grasartigen Unkräutern, verwendet werden können (vgl. DT—OS 2 328 340). Diese Verbindungen sind in ihrer Selektivität jedoch nicht immer befriedigend. Weiterhin sind bestimmte Halbäther von cyclischen 1,2-Diolen bekannt (vgl. FR—PS 1 176 120). Diese können als Zwischenprodukte für die Herstellung von Kunststoffen und Weichmachern verwendet werden; eine herbizide Wirkung dieser Stoffe ist dagegens nicht bekannt.

Es wurden neue Benzyläther von cyclischen 1,2-Diolen der Formel

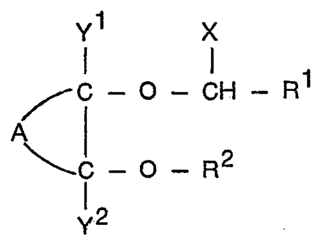

(I),

in welcher

$R_1$ für Phenyl steht, welches durch einen oder mehrere der folgenden Reste substituiert sein kann: Halogen (insbesondere Fluor, Chlor oder Brom); Alkyl, Alkoxy und Alkylthio mit jeweils 1 bis 4 Kohlenstoffatomen; Halogenalkyl, Halogenalkoxy und Halogenalkylthio mit jeweils bis zu 4 Kohlenstoffatomen und bis zu 5 Halogenatomen (insbesondere mit bis zu 2 Kohlenstoff- und bis zu 3 gleichen oder verschiedenen Halogenatomen, wobei als Halogene insbesondere Fluor, Chlor und Brom stehen); durch (weiteres) Phenyl, Phenoxy oder Phenoxycarbonyl, welche jeweils durch Halogen (insbesondere Fluor, Chlor oder Brom), Alkyl oder Alkoxy mit jeweils 1 bis 2 Kohlenstoffatomen sowie durch Halogenalkyl mit bis zu 2 Kohlenstoff- und bis zu 3 gleichen oder verschiedenen Halogenatomen (wie insbesondere Fluor und Chlor) substituiert sein können; durch Alkoxycarbonyl mit 1 bis 4 kohlenstoffatomen im Alkylteil; Alkylamino und Dialkylamino mit 1 bis 4 Kohlenstoffatomen in jedem Alkylrest; durch die Methylen-dioxy-Gruppe sowie durch den Tri-, Tetra- oder Pentamethylen-Rest;

$R^2$ für geradkettiges oder verzweigtes Alkyl mit 1 bis 8 Kohlenstoffatomen; Alkenyl und Alkinyl mit jeweils 2 bis 4 Kohlenstoffatomen; Halogenalkyl mit bis zu 2 Kohlenstoff- und bis zu 3 gleichen oder verschiedenen Halogenatomen (wie insbesondere Fluor und Chlor) sowie für die Gruppierung —CHX—$R^1$ steht;

X für Wasserstoff; geradkettiges oder verzweigtes Alkyl mit 1 bis 4 Kohlenstoffatomen; Alkenyl und Alkinyl mit jeweils 2 bis 4 Kohlenstoffatomen; Halogenalkyl mit bis zu 3 Kohlenstoff- und bis zu 3 gleichen oder verschiedenen Halogenatomen (wie insbesondere Fluor und Chlor) sowie für Phenyl, welches durch Halogen, Alkyl oder Alkoxy mit jeweils 1 oder 2 Kohlenstoffatomen sowie Halogenalkyl mit bis zu 2 Kohlenstoff- und bis zu drei gleichen oder verschiedenen Halogenatomen (wie insbesondere Fluor und Chlor) substituiert sein kann, steht;

$Y_1$ und $Y_2$ gleich oder verschieden sind und für Wasserstoff; geradkettiges oder verzweigtes Alkyl mit 1 bis 6 Kohlenstoffatomen sowie für Phenyl steht, welches durch die folgenden Reste substituiert sein kann; Halogen, Alkyl und Alkoxy mit jeweils 1 bis 4 Kohlenstoffatomen; Halogenalkyl, Halogenalkoxy oder Halogenalkylthio mit jeweils bis zu 4 Kohlenstoffatomen und bis zu 5 Halogenatomen (vorzugsweise mit bis zu 2 Kohlenstoff- und bis zu 3 gleichen oder verschiedenen Halogenatomen, insbesondere Fluor, Chlor und Brom); und

A für eine zwei- bis sechsgliedrige gesättigte oder ungesättigte Alkylen-Brücke steht, die durch einen oder mehrere der folgenden Reste substituiert sein kann; durch Halogen (insbesondere Chlor oder Brom); geradkettiges oder verzweigtes Alkyl mit 1 bis 6 Kohlenstoffatomen; durch eine Di-, Tri-, Tetra- oder Pentamethylenbrücke oder Phenyl oder durch Halogen, Alkyl und Alkoxy mit jeweils 1 bis 4 Kohlenstoffatomen oder durch Halogenalkyl, Halogenalkoxy und Halogenalkylthio mit jeweils bis zu 4 Kohlenstoffatomen und bis zu 5 Halogenatomen (insbesondere mit bis zu 2 Kohlenstoff- und bis zu 3 gleichen oder verschiededen Halogenatomen, wobei als Halogene insbesondere Fluor, Chlor und Brom stehen) substituiertes Phenyl),
aufgefunden.

Diese neuen Benzyläther von cyclischen 1,2-Diolen weisen starke herbizide, insbesondere selektiv-herbizide Eigenschaften auf.

Die Verbindungen der Formel (I) können jeweils als geometrische Isomere, d.h. als cis- und als trans-Isomere bzw. als Gemische dieser beiden Isomeren vorliegen. Darüber hinaus liegen diese Verbindungen größtenteils jeweils auch als optische Isomere vor. Sämtliche Isomeren sind Gegenstand der Erfindung.

Weiterhin wurde gefunden, daß man die Benzyläther von cyclischen 1,2-Diolen der Formel (I) erhält, wenn man cyclische 1,2-Diole der Formel

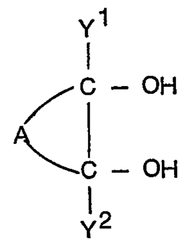

(II)

in welcher

A, $Y^1$ und Y die oben angegebene Bedeutung haben,

mit Verbindungen der Formel

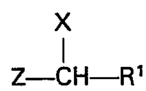

(III)

in welcher

$R^1$ und X die oben angegebene Bedeutung haben und

Z für Halogen (insbesondere Chlor, Brom oder Jod), den Mesylate- oder Tosylat-Rest steht,

in Gegenwart einer starken Base und in Gegenwart eines Verdünnungsmittels umsetzt und gegebenenfalls die entstehenden Benzyläther der Formel

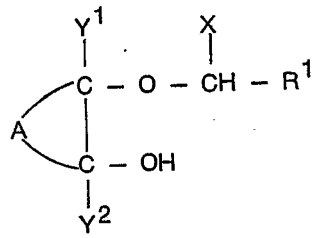

(IV)

in welcher

A, $R^1$, X

$Y^1$ und $Y^2$ die oben angegebene Bedeutung haben,

mit Verbindungen der Formel

$$Z—R^3 \qquad (V)$$

in welcher

Z die oben angeführte Bedeutung hat und

$R^3$ für geradkettiges oder verzweigtes Alkyl mit 1 bis 8 Kohlenstoffatomen, Alkenyl oder Alkinyl mit jeweils 2 bis 4 Kohlenstoffatomen, Halogenalkyl mit bis zu 2 Kohlenstoff- und bis zu 3 gleichen oder verschiedenen Halogenatomen (wie insbesondere Fluor und Chlor) oder für die Gruppierung —CHX—$R^1$ steht, wobei $R^1$ und X die oben angegebene Bedeutung haben,

in Gegenwart einer starken Base und in Gegenwart eines Verdünnungsmittels umsetzt (Verfahrensweg A).

In manchen Fällen erweist es sich als vorteilhaft, die cyclischen 1,2-Diole der Formel (II) zuerst mit Verbindungen der Formel (V) umzusetzen und die entstehenden Aether der Formel

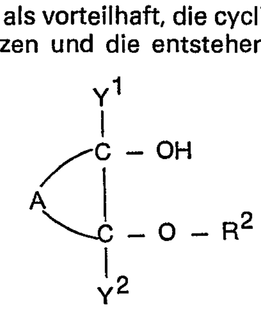

(VI)

in welcher

A, $R^2$, $Y^1$ und $Y^2$ die oben angegebene Bedeutung haben,

anschließend mit Verbindungen der Formel (III) umzusetzen, jeweils unter den bei Verfahrensweg (A) angegebenen Bedingungen (Verfahrensweg B).

Ueberasschenderweise sind die erfindungsgemäßen Benzyläther von cyclischen 1,2-Diolen den vorbekannten Gräserbekämpfungsmitteln, wie beispielsweise 2-Aethyl-6-methyll-N-(1′-methyl-2′-methoxyäthyl)-chloracetanilid, in der herbiziden Wirkung deutlich überlegen und zeigen außerdem eine ausgezeichnete Selektivität in wichtigen Kulturpflanzen. Die erfindungsgemäßen Wirkstoffe stellen somit eine wesentliche Bereicherung der herbiziden Mittel, insbesondere der Gräserherbizide, dar.

Verwendet man nach Verfahrensvariante (A) cis-1,2-Cyclobutandiol, 2-Fluorbenzylchlorid und Methyljodid als Ausgangsstoffe sowie Natriumhydroxid als starke Base, so kann der Reaktionsablauf durch das folgende Formelschema wiedergegeben werden:

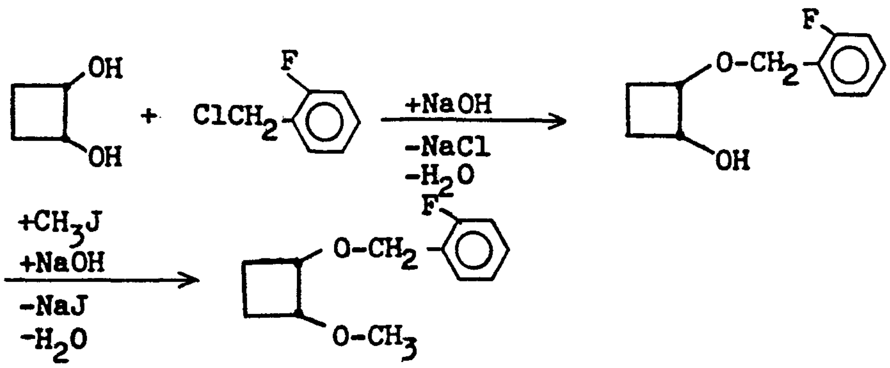


Verwendet man nach Verfahrensweg (B) trans-1,2-Dimethyl-cyclohexan-1,2-diol, Athylbromid und 2,4-Dichlorbenzylchlorid als Ausgangsstoffe sowie Kaliumhydroxid als starke Base, so kann der Reaktionsablauf durch das folgende Formelschema wiedergegeben werden:

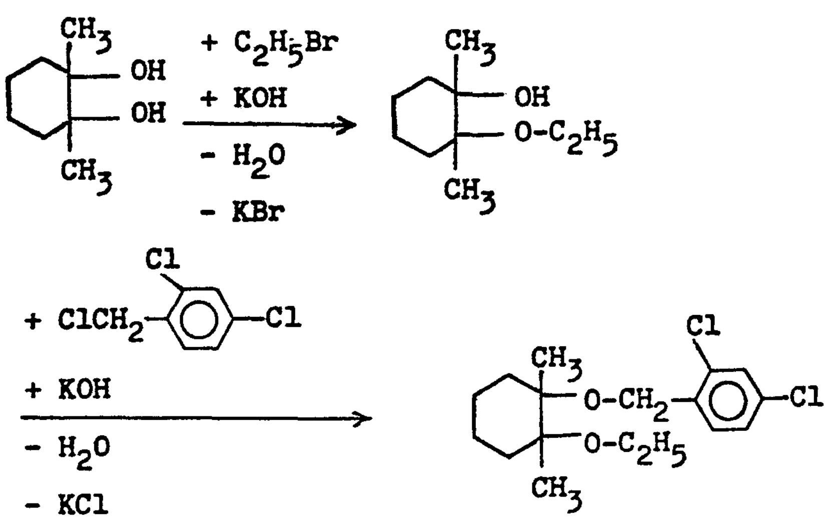


Die cyclischen 1,2-Diole der Formel (II) sind bekannt (vgl. Tetrahedron Letters *1972,* 857—860; F. D. Gunstone in Advances in Organic Chemistry, Methods and Results, Vol. 1 Interscience Publishers, New York (1960); J. Am. Chem. Soc. *56,* 1993 (1934)) oder lassen sich nach bekannten Methoden herstellen. Man erhält sie z.B., indem man 1,2-Bis-(trimethylsiloxy)-cycloalkene der Formel

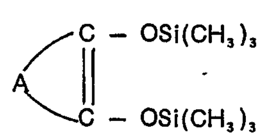

(VII)

in welcher

A die oben angegebene Bedeutung hat,

(a) mit Wasserstoff in Gegenwart eines Katalysators, wie beispielsweise Palladium/Kohlenstoff, bei einer Temperatur von 20—80°C und einem Druck von 80—100 at hydriert und die entstehenden cis-1,2-Bis-(trimethylsiloxy)-cycloalkane der Formel

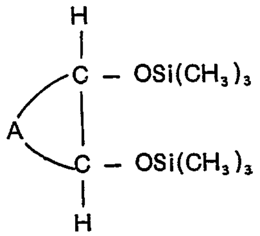

(VIII)

in welcher

A die oben angegebene Bedeutung hat,

in üblicher Weise hydrolisiert (vgl. Tetrahedron Letters *1972,* 857—860) oder

(b) einer sauren Hydrolyse oder Alkoholyse unterwirft (vgl.K. Rühlmann, Synthesis *1971,* 236) und die entstehenden cyclischen α-Hydroxy-ketone der Formel

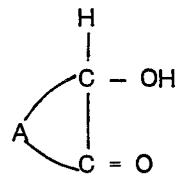

(IX)

in welcher

A die oben angegebene Bedeutung hat,

in üblicher Weise mit komplexen Hydriden, wie beispielsweise Natriumborhydrid, in Gegenwart eines polaren organischen Lösungsmittels, wie beispielsweise Alkohole, bei Temperaturen zwischen 0 bis 30°C reduziert oder mit Grignard Reagenzien in Gegenwart wasserfreier Aether als Verdünnungsmittel bei Temperaturen zwischen 30 und 60°C umsetzt (vgl. J. Am. Chem. Soc. *56,* 1993 (1934)).

Die cyclischen 1,2-Diole der Formel (II) können auch erholten werden, wenn man

(c) Cycloalkene der Formel

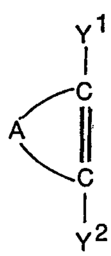

(X)

in welcher

A, $Y^1$ und $Y^2$ die oben angegebene Bedeutung haben,

in üblicher Weise mit Kaliumpermanganat oder Osmiumtetroxid bei Temperaturen zwischen −80°C und Raumtemperatur oxidiert (vgl. F. D. Gunstone in Advances in Organic Chemistry, Methods and Results, Vol. 1, Interscience Publishers, New York (1960)). Die Oxidation kann auch phasentransferkatalysiert durchgeführt wurden (vgl. Tetrahedron Letters *1972,* 4907—8).

Die als Ausgangsstoffe zu verwendenden 1,2-Bis-(trimethylsiloxy)-cycloalkene der Formel (VII) sind bekannt (vgl. K. Rühlmann, Synthesis *1971,* 236) bzw. lassen sich nach den dort beschriebenen Verfahren herstellen, z.B. durch Umsetzung von Dicarbonsäureestern mit Natrium in inerten Lösungsmitteln unter Zusatz von Trimethylchlorsilan.

Die cyclischen α-Hydroxy-ketone der Formel (IX) können auch erhalten werden, indem man entsprechende Epoxide mit Dimethylsulfoxid, gegebenenfalls in Gegenwart eines Katalysators, wie beispielsweise Bortrifluorid, oxidiert (vgl. J. Org. Chem. *26,* 1681 (1961)).

Die als Ausgangsstoffe zu verwendenden Cycloalkene der Formel (X) sind allgemein bekannte Verbindungen der organischen Chemie.

Als Beispiele für die als Ausgangsstoffe zu verwendenden cyclischen 1,2-Diole der Formel (II) seien genannt:

Cyclobutan - 1,2 - diol, Cyclopentan - 1,2 - diol, Cyclohexan - 1,2 - diol, Cycloheptan - 1,2 - diol, Cyclooctan - 1,2 - diol, 1 - Methyl - cyclobutan - 1,2 - diol, 1 - Methyl - cyclopentan - 1,2 -

diol, 1 - Methyl - cyclohexan - 1,2 - diol, 1 - Methyl - cycloheptan - 1,2 - diol, 1 - Methyl - cyclooctan - 1,2 - diol, 1 - Aethyl - cyclobutan - 1,2 - diol, 1 - Aethyl - cyclopentan - 1,2 - diol, 1 - Aethyl - cyclohexan - 1,2 - diol, 3,4 - Tetramethylen - cyclobutan - 1,2 - diol, Cyclohex - 4 - en - 1,2 - diol, 1,2 - Dimethyl - cyclobutan - 1,2 - diol, 1,2 - Dimethyl - cyclohexan - 1,2 - diol, 1,2 - Dimethyl - cycloheptan - 1,2 - diol, 1,2 - Dimethyl - cyclooctan - 1,2 - diol, 1 - Phenyl - cyclobutan - 1,2 - diol, 1 - Phenyl - cyclopentan - 1,2 - diol, 1 - Phenyl - cyclohexan - 1,2 - diol, 1 - Phenyl - cycloheptan - 1,2 - diol, 3 - Methyl - cyclobutan - 1,2 - diol, 3 - Aethyl - cyclobutan - 1,2 - diol, 3 - Isopropyl - cyclobutan - 1,2 - diol, 3 - tert. - Butyl - cyclobutan - 1,2 - diol, 3,3 - Dimethyl - cyclobutan - 1,2 - diol.

Die Ausgangsstoffe der Formel (III) sind allgemein bekannte Verbindungen der organischen Chemie. Als Beispiele seien genannt:

Benzylchlorid, Benzylbromid, Benzyljodid, Benzylmesylat, Benzyltosylat, 2-Fluorbenzyljodid, 2-Fluorbenzylbromid, 3-Fluorbenzylbromid, 4-Fluorbenzylbromid, 3-Chlorbenzylchlorid, 4-Chlorbenzylchlorid, 2-Brombenzylchlorid, 3-Brombenzylchlorid, 4-Brombenzylchlorid, 2-Methylbenzylchlorid, 3-Methylbenzylchlorid, 4-Methylbenzylchlorid, 2-Methoxybenzylchlorid, 3-Methoxybenzylchlorid, 4-Methoxybenzylchlorid, 2-Trifluormethylbenzylchlorid, 3-Trifluormethylbenzylchlorid, 4-Trifluormethylbenzylchlorid, 4-Phenylbenzylchlorid, 2,6-Difluorbenzylchlorid, 2,6-Dichlorbenzylchlorid, 2,4-Dichlorbenzylchlorid, 3,4-Dichlorbenzylchlorid, 2,5-Dichlorbenzylchlorid, 2,6-Dimethylbenzylchlorid, 2,4-Dimethylbenzylbromid, 3,4-Dimethylbenzylchlorid, 2,3-Dimethylbenzylchlorid, 3,4-Dioxymethylenbenzylchlorid, 2,6-Chlorfluorbenzylchlorid, 2-Fluor-5-chlorbenzylbromid, 2-Fluor-4-chlorbenzylbromid, 3-Chlor-4-fluorbenzylbromid, 3,4-Tetramethylenbenzylchlorid, 2-Methyl-6-chlorbenzylchlorid, 2-Methyl-6-fluorbenzylchlorid, 2-Fluor-3-methylbenzylchlorid, 2-Fluor-4-methylbenzylchlorid, 2-Fluor-5-methylbenzylchlorid, 2-Methyl-3-chlorbenzylchlorid, 2-Methyl-4-chlorbenzylchlorid, 2-Methyl-5-chlorbenzylchlorid, 2,4,5-Trichlorbenzylbromid, 2,4,6-Trichlorbenzylbromid, Diphenylmethylbromid, 1-Brom-1-phenyläthan, 1-Brom-1-(2-methylphenyl)-äthan.

Die Ausgangsstoffe der Formel (V) sind allgemein bekannte Verbindungen der organischen Chemie. Als Beispiele seien genannt:

Methylchlorid, Methylbromid, Methyljodid, Aethylbromid, nl-Propylbromid, Isopropylbromid, tert.-Butyljodid, Allylbromid, Allyljodid, Vinylbromid, Buten-2-yl-chlorid, Propargylchlorid sowie die bereits genannten Beispiele der Verbindungen der Formel (III).

Die möglichen Zwischenprodukte der Formel (VI) sind teilweise bekannt (vgl. J. Chem. Soc. (London) *1964,* 2846—8) und können auch nach den dort angegebenen Verfahren hergestellt werden. Man erhält sie ebenfalls, wenn man entsprechende o-Alkoxyphenole in üblicher Weise in Gegenwart eines Katalysators hydriert.

Für die erfindungsgemäße Umsetzung kommen als Verdünnungsmittel vorzugsweise inerte organische Lösungsmittel infrage. Hierzu gehören vorzugsweise Aether, wie Diäthyläther, Tetrahydrofuran oder Dioxan, aromatische Kohlenwasserstoffe, wie Benzol oder Toluol, in einzelnen Fällen auch chlorierte Kohlenwasserstoffe, wie Chloroform, Methylenchlorid oder Tetrachlorkohlenstoff sowie Dimethylsulfoxid.

Die erfindungsgemäß Umsetzung wird in Gegenwart einer starken Base durchgeführt. Dazu gehören vorzugsweise Alkalimetallhydride, -amide, -hydroxide und -carbonate, wie beispielsweise Natriumhydrid, Natriumamid, Natriumhydroxid, Kaliumhydroxid und Kaliumcarbonat.

Die Reaktionstemperaturen können bei dem erfindungsgemäßen Verfahren in einem größeren Bereich veriiert werden. Im allgemeinen arbeitet man zwischen 0 und 120°C, vorzugsweise bei 20 bis 100°C.

Bei der Durchführung des erfindungsgemäßen Verfahrens arbeitet man vorzugsweise in molaren Mengen, wobei es gegebenenfalls vorteilhaft sein kann, jeweils die Base und die Verbindungen der Formel (V) in einem Ueberschuß bis zu 1,5 Mol einzusetzen. Zur Isolierung der Endprodukte wird das Reaktionsgemisch mit Wasser versetzt, gegebenenfalls ein organisches Solvens zugegeben, die organische Phase abgetrennt und in üblicher Weise aufgearbeitet und gereinigt.

Nach einer bevorzugten Ausführungsform wird die erfindungsgemäß Umsetzung in einem Zweiphasensystem, wie z.B. wässrige Natron- oder Kalilauge/Toluol oder Methylenchlorid, unter Zusatz eines Phasen-Transfer-Katalysators, zum Beispiel Ammoniumverbindungen wie Triäthyl-benzylammoniumchlorid, Tetra-n-butyl-ammoniumchlorid oder -bromid, durchgeführt.

Als Beispiel für besonders wirksame Vertreter der erfindungsgemäßen Wirkstoffe seien außer den Herstellungsbeispielen und den Beispielen der Tabelle 1 genannt:

1,2-Bis-(2-fluorbenzyloxy)-cyclobutan
1-(2-Fluorbenzyloxy)-2-methoxy-2-methyl-cyclobutan
1-(2-Fluorbenzyloxy)-2-methoxy-2-methyl-cyclopentan
1-(2-Fluorbenzyloxy)-2-methoxy-2-methyl-cyclohexan
1-(2-Fluorbenzyloxy)-2-methoxy-2-methyl-cycloheptan
1-(2-Fluorbenzyloxy)-2-methoxy-2-methyl-cyclooctan
1,2-Bis-(2-fluorbenzyloxy)-1-äthyl-cyclopentan
1-(2-Fluorbenzyloxy)-2-äthyl-2-methoxy-cyclopentan.

Die erfindungsgemäßen Wirkstoffe beeinflussen das Pflanzenwachstum und können deshalb als

Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

*Dikotyle Unkräuter der Gattungen:* Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

*Dicotyle Kulturen der Gattungen:* Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cuburbita.

*Monokotyle Unkräuter der Gattungen:* Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Shpenoclea, Dactyloctenium, Agrostis, Alopecurus, Apera.

*Monokotyle Kulturen der Gattungen:* Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe können vorzugsweise zur selektiven Unkraut- und besondere Ungräserbekämpfung in verschiedenen Kulturen eingesetzt werden. Mit den erfindungsgemäßen Wirkstoffen ist es — im Gegensatz zu den als Gräserherbizide bekannten Chloracetaniliden — möglich, die schwer bekämpfbaren Ungräser Alopecurus oder Poa annua gleichzeitig mit anderen Schadgräsern, z.B. Digitaria, Echinochloa, Panicum und/oder Setaria in Kulturen, wie Zuckerrüben, Sojabohnen, Baumwolle, Raps, Erdnüssen, Gemüsearten, Mais und Reis erfolgreich zu bekämpfen.

Die Wirkstoffe können in die üblichen Formulierungen übergeführt werden, wie Lösungen, Emulsionen, Spritzpulver, Suspensionen, Pulver, Pasten, lösliche Pulver, Granulate, Suspensions-Emulsionskonzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehle, Kokosnußschalen, Maiskolben und Tabakstengel; als Emulgiermittel; nichtionogene und anionische Emulgatoren, wie Polyoxyäthylen-Fettsäure-Ester, Polyoxyäthylen-Fettalkohol-Äther, z.B. Alkylaryl-polyglykol-äther, Alkylsulfonate, Alkylsulfate, Arylsulfonate sowie Eiweißhydrolysate; als Dispergiermittel: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polvinylacetat.

Es können Farbstoffe wie anorganische Pigmente, z.B. Eisenoxid, Titanoxid, Ferrocyanblau und organische Farbstoffe, wie Alizarin-, Azo-Metallphthalocyaninfarbstoffe und Spurennährstoffe wie Salze von Eisen, Mangan, Bor, Kupfer, Kobalt, Molybdän und Zink werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können als solche oder in ihren Formulierungen auch in Mischung mit bekannten Herbiziden zur Unkrautbekämpfung Verwendung finden, wobei

Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewandt werden. Die Anwendung geschieht in üblicher Weise, z.B. durch Gießen, Spritzen, Sprühen, Stäuben oder Streuen.

Die erfindungsgemäßen Wirkstoffe können sowohl nach als auch insbesondere vor Auflaufen der Pflanzen appliziert werden. Sie können auch vor der Saat in den Boden eingearbeitet werden.

Die aufgewandte Wirkstoffmenge kann in größeren Bereichen schwanken. Sie hängt im wesentlichen von der Art des gewünschten Effekts ab. Im allgemeinen liegen die Aufwandmengen zwischen 0,1 und 10 kg Wirkstoff pro ha, vorzugsweise zwischen 0,1 und 5 kg/ha.

Beispiel A

Pre-emergence-Test

Lösungsmittel: 5 Gewichtsteile Aceton

Emulgator: 1 Gewichtsteil Alkylarylpolyglycoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Hierbei bedeuten 0% keine Wirkung (wie unbehandelte Kontrolle), 100% bedeuten totale Vernichtung.

Bei diesem Test zeigen z.B. die folgenden Verbindungen der Herstellungsbeispiele eine ausgezeichnete Wirkung, welche der Wirkung der aus dem Stand der Technik bekannten Verbindungen gleicher Wirkungsrichtung deutlich überlegen ist: 1, 6, 30, 31, 61.

Herstellungsbeispiele

Beispiel 1

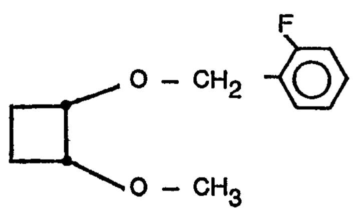

396,0g (4,5 Mol) cis-1,2-Cyclobutandiol und 650,3g (4,5 Mol) 2-Fluorbenzylchlorid werden in 2,5 l Dimethylsulfoxid gelöst und bei Raumtemperatur unter Eiskühlung potionsweise mit 270g (6,75 Mol) Natriumhydroxidpulver versetzt. Man läßt 6 Stunden bei Raumtemperatur rühren und gibt dann unter Kühlung 958,5g (6,75 Mol) Methyljodid und portionsweise 270g (6,75 Mol) Natriumhydroxidpulver hinzu. Danach läßt man erneut 6 Stunden bei Raumtemperatur rühren und gibt das Reaktionsgemisch auf 10 l Eiswasser. Man extrahiert dreimal mit je 5 l Petroläther, trocknet die vereinigten organischen Phasen über Natriumsulfat und engt durch Abdestillieren des Lösungsmittels ein. Man erhält 840g (88,9% der Theorie) cis-1-(2-Fluorbenzyloxy)-2-methoxy-cyclobutan vom Brechungsindex $n_D^{21} = 1,4948$.

Die Herstellung des Ausgangsproduktes cis-1,2-Cyclobutandiol erfolgt durch katalytische Hydrierung des 1,2-Bis-(trimethylsiloxy)-cyclobut-1-ens zum cis-1,2-Bis-(trimethylsiloxy)-cyclobutan und dessen Hydrolyse gemäß Tetrahedron Letters *9*, 857—860 (1972).

Beispiel 2

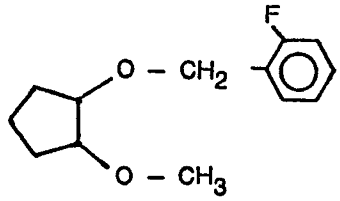

6,3g (0,03 Mol) cis-1-(2-Fluorbenzyloxy)-cyclopentan-2-ol in 100 ml absolutem Dioxan werden zu

einer Suspension von 0,72g (0,03 Mol) Natriumhydrid (0,9g 80%iges Natriumhydrid in Toluol) in 100ml absolutem Dioxan bei 5°C unter Stickstoff getropft. Nach 30-minütigem Rühren bei Raumtemperatur wird 45 Minuten auf 70°C erwärmt, abgekühlt, 4,26g (0,03 Mol) Methyljodid in Dioxan zugetropft und anschließend 4 Stunden zum Sieden erhitzt. Nach dem Abkühlen auf Raumtemperatur werden 5 ml Methanol zugetropft und die Suspension am Rotationsverdampfer eingeengt. Der Rückstand wird in Wasser aufgenommen und nochmals mit Methylenchlorid extrahiert. Die vereinigten Methylenchloridphasen werden über Natriumsulfat getrocknet, filtriert und das Lösungsmittel am Rotationsverdampfer abgezogen. Der ölige Rückstand wird destilliert. Man erhält 5 g (75% der Theorie) cis-1-(2-Fluorbenzyloxy)-2-methoxy-cyclopentan vom Siedepunkt 90°C/0,1 mm.

*Herstellung der Ausgangsprodukte*

a)

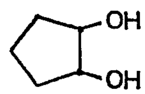

Eine Lösung von 1000ml tert.-Butanol, 200ml Wasser, 500g zerstoßenem Eis und 7g (0,1 Mol) Cyclopenten wird auf —10°C abgekühlt. Dazu wird innerhalb von 5 Minuten eine auf 0°C gekühlte Lösung von 20,5g (0,13 Mol) Kaliumpermanganat und 4g (0,1 Mol) Natriumhydroxid gegeben und das Gemisch 5 Minuten bei 0°C gerührt. Dann wird eine Lösung von 0,05 Mol Natriumsulfit in 50ml Wasser zugetropft, der Braunstein abfiltriert und tert.-Butanol abdestilliert. Anschließend wird die Lösung am Rotationsverdampfer auf ca. 200ml eingeengt und mehrmals mit Aether extrahiert. Die vereinigten Aetherphasen werden über Natriumsulfat getrocknet, filtriert, das Lösungsmittel abgezogen und der Rückstand destilliert. Man erhält 3.4g (33% der Theorie) cis-1,2-Cyclopentandiol vom Siedepunkt 114°C/12mm.

b)

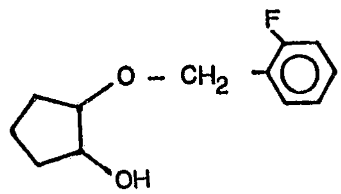

Zu einer Suspension von 4,8g (0,2 Mol) Natriumhydrid (6,0 g 80%iges Natriumhydrid in Toluol) in 200ml Dioxan werden bei 5°C unter einer Stickstoffathmosphäre 19,4g (0,19 Mol) cis-1,2-Cyclopentandiol unter Rühren zugetropft. Nach beendeter Wasserstoffentweichung wird das Gemisch 45 Minuten auf 70°C erwärmt, dann auf Raumtemperatur gebracht und mit 359g (0,19 Mol) 2-Fluorbenzylbromid in absolutem Dioxan versetzt. Nach 30-minütigem Rühren wird noch 4 Stunden zum Sieden erhitzt, abgekühlt und circa 10ml Methanol zugesetzt. Das Gemisch wird am Rotationsverdampfer zur Trockne eingeengt, der Rückstand in Wasser aufgenommen und mit Methylenchlorid extrahiert. Die organische Phase wird über Natriumsulfat getrocknet, filtriert und das Lösungsmittel am Rotationsverdampfer abgezogen. Der ölige Rückstand wird im Oelpumpenvakuum destilliert. Man erhält 18,4g (46% der Theorie) cis-1-(2-Fluorbenzyloxy)-cyclopentan-2-ol vom Siedepunkt 95—100°C/0,1mm.

Beispiel 3

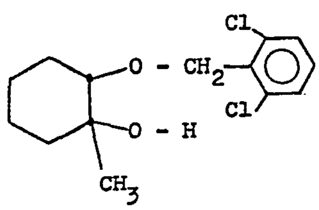

und

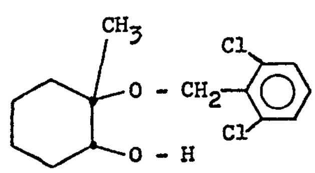

Zu einer Suspension von 0.72g (0,03 Mol) Natriumhydrid (0,9g 80%iges Natriumhydrid in Toluol) in 100 ml absolutem Dioxan wird in einem Stickstoffstrom under Kühlung eine Lösung von 3,9g (0,03 Mol) cis-1-Methyl-cyclohexan-1,2-diol getropft. Nach Beendigung der Wasserstoffentwicklung wird das Gemisch 30 Minuten auf 70°C erwärmt und nach den Abkühlen 5,9g (0,03 Mol) 2,6-Dichlorbenzylchlorid zugetropft. Nach 30-minütigem Rühren bei Raumtemperatur wird die Mischung 4 Stunden unter Rückfluß erhitzt, nach dem Abkühlen 5 ml Methanol zugegeben und das Gemisch am Rotationsverdampfer zur Trockne eingeengt. Der Rückstand wird in Wasser aufgenommen und nochmals mit Methylenchlorid extrahiert. Die vereinigten organischen Phasen werden über Natriumsulfat getrocknet, filtriert und das Lösungsmittel am Rotationsverdampfer abgezogen. Der

oelige Rückstand wird destilliert. Man erhält 7g (81% der Theorie) als Gemisch von cis-1-(2,6-Dichlorbenzyloxy)-2(bzw. 1)-methyl-cyclohexan-2-ol vom Siedepunkt 135—138°C/0,1 mm.

*Herstellung des Ausgangsproduktes*

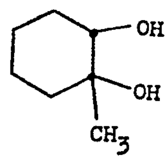

Zu einer Lösung von 116,2g (0,7 Mol) Methylmagnesiumjodid in Aether werden 38,8g (0,34 Mol) 2-Hydroxycyclohexanon in Aether unter Rühren getropft. Nach beendeter Reaktion wird die Suspension auf eine Mischung aus Wasser, Aether und verdünnter Schwefelsäure gegossen, die Aetherphase abgetrennt und die wässrige Phase mehrmals mit Aether extrahiert. Die vereinigten Aetherextrakte werden über Natriumsulfat getrocknet, das Lösungsmittel am Rotationsverdampfer abgezogen und der Rückstand destilliert. Man erhält 21,2g (48% der Theorie) 1-Methylcyclohexan-1,2-diol vom Siedepunkt 75°C/0,2mm, das zu >90% in der cis-Form vorliegt.

*Herstellung des Ausgangsproduktes*

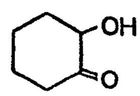

98g (1 Mol) Cyclohexenoxid in 200 ml absolutem Dimethylsulfoxid werden mit 0,4 ml Bortrifluorid-Aetherat versetzt und unter Rühren 22 Stunden auf 120°C erhitzt. Nach 10 Stunden bzw. 20 Stunden erfolgt weiterer Zusatz von je 0,2 ml Bortrifluorid-Aetherat. Nach dem Abkühlen wird das Rekationsgemisch auf Eis geschüttet und die wässrige Lösung mit Chloroform extrahiert. Die organische Phase wird über Natriumsulfat getrocknet, filtriert und das Lösungsmittel am Rotationsverdampfer abgezogen. Der Rückstand wird im Wasserstrahlvakuum destilliert. Man erhält 88,4g (68% der Theorie) 2-Hydroxycyclohexanon vom Siedepunkt 83—85°C/12mm.

Beispiel 4

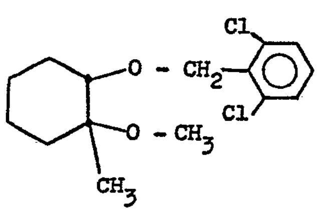

und

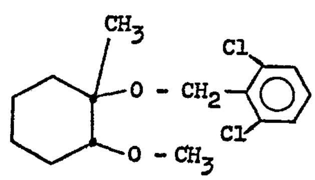

87g (0,03 Mol) eines Gemisches von cis-1-(2,6-Dichlorbenzyloxy)-2(bzw.1)-methyl-cyclohexan-2-ol (Beispiel 4) werden in 100 ml absolutem Dioxan unter Stickstoffatmosphäre bei 5°C zu einer Suspension von 0,72g (0,03 Mol) Natriumhydrid (0,9g 80%iges Natriumhydrid in Toluol) in 100 ml absolutem Dioxan getropft. Nach 30-minütigem Rühren bei Raumtemperatur wird die Mischung 45 Minuten auf 70°C erwärmt und nach dem Abkühlen mit 4,26g (0,03 Mol) Methyljodid versetzt. Es wird 30 Minuten bei Raumtemperatur gerührt, danach 4 Stunden zum Sieden erhitzt. Nach dem Abkühlen werden ca. 5 ml Methanol zugegeben und das Gemisch am Rotationsverdampfer eingeengt. Der Rückstand wird in Wasser aufgenommen und die wäßrige Phase mehrmals mit Methylenchlorid extrahiert. Die vereinigten organischen Phasen werden über Natriumsulfat getrocknet, filtriert, das Lösungsmittel am Rotationsverdampfer abgezogen und der ölige Rückstand destilliert. Man erhält 5g (55% der Theorie) als Gemisch von cis-1-(2,6-Dichlorbenzyloxy)-2-methoxy-2(bzw. 1)-methyl-cyclohexan vom Siedepunkt 131°C/0,1 mm.

Beispiel 5

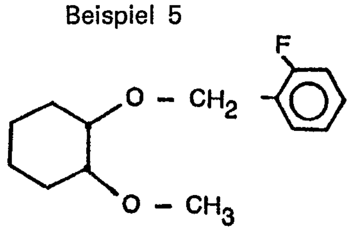

Zu 1672g (12,8 Mol) cis-2-Methoxy-cyclohexanol und 1835g (12,8 Mol) 2-Fluorbenzylchlorid in 10 l Dimethylsulfoxid gibt man unter Wasserkühlung potionsweise 772g (19,3 Mol) Natriumhydroxid-Pulver. Man läßt 2 Stunden bei 35°C und über Nacht bei 45°C rühren. Danach wird auf 18°C abgekühlt, mit 10 l Petroläther versetzt und auf 10 l Eiswasser gegeben. Die organische Phase wird abgetrennt, mit Wasser ausgeschüttelt, über Natriumsulfat getrocknet, filtriert und eingeengt. Der ölige Rückstand wird destilliert. Man erhält 2685g (87,5% der Theorie) cis-1-(2-Fluorbenzyloxy)-2-methoxy-cyclohexan vom Siedepunkt 150°C/0,2 mm.

Das Ausgangsprodukt cis-2-Methoxy-cyclohexanol wird entsprechend J. Chem. Soc. (London) *1964*, 2846—8 hergestellt.

In analoger Weise werden die in der nachfolgenden Tabelle 1 aufgeführten Verbindungen hergestellt.

TABELLE 1

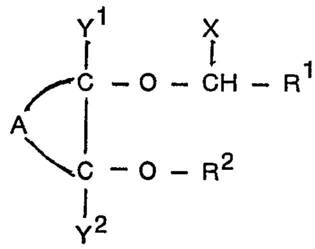

(I)

| Beispiel Nr. | $R^1$ | $R^2$ | X | $Y^1$ | $Y^2$ | A | Kp(°C)/mm Hg-Säule |
|---|---|---|---|---|---|---|---|
| 6 | 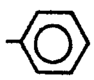 | $CH_3$ | H | H | H | $-(CH_2)_4-$ | 155 /20 (trans) |
| 7 | 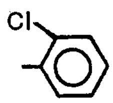 | $CH_3$ | H | H | H | $-(CH_2)_4-$ | 180 /20 (trans) |
| 8 | 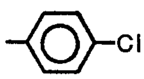 | $CH_3$ | H | H | H | $-(CH_2)_4-$ | 142 /0,2 (trans) |
| 9 | 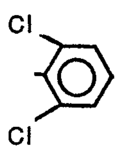 | $CH_3$ | H | H | H | $-(CH_2)_4-$ | 125 /0,1 (trans) |
| 10 | 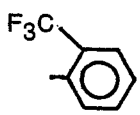 | $CH_3$ | H | H | H | $-(CH_2)_4-$ | 125 /0,1 (trans) |
| 11 | 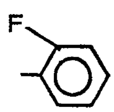 | $CH_3$ | H | H | H | $-(CH_2)_4-$ | 120 /0,2 (trans) |
| 12 | 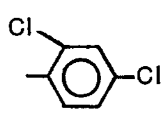 | $CH_3$ | H | H | H | $-(CH_2)_4-$ | 200 /0,5 (trans) |
| 13 | 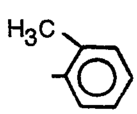 | $CH_3$ | H | H | H | $-(CH_2)_3-$ | 95 /0,1 (trans) |
| 14 | 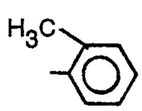 | $CH_3$ | H | H | H | $-(CH_2)_4-$ | 115 /0,1 (trans) |

TABELLE 1 (Fortsetzung)

| Beispiel Nr. | $R^1$ | $R^2$ | X | $Y^1$ | $Y^2$ | A | Kp(°C)/mm Hg–Säule |
|---|---|---|---|---|---|---|---|
| 15 | 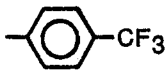 | $CH_3$ | H | H | H | $-(CH_2)_3-$ | 85 /0,1 (trans) |
| 16 | 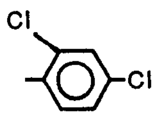 | $CH_3$ | H | H | H | $-(CH_2)_3-$ | 110 /0,1 (trans) |
| 17 | 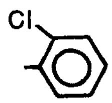 | $CH_3$ | H | H | H | $-(CH_2)_3-$ | 115–25 /0,2 (trans) |
| 18 | 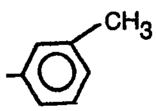 | $CH_3$ | H | H | H | $-(CH_2)_4-$ | 125 /0,1 (trans) |
| 19 | 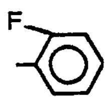 | $CH_3$ | H | H | H | $-(CH_2)_3-$ | 85 /0,2 (trans) |
| 20 | 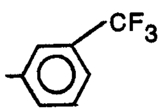 | $CH_3$ | H | H | H | $-(CH_2)_4-$ | 90 /0,05 (trans) |
| 21 | 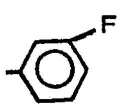 | $CH_3$ | H | H | H | $-(CH_2)_4-$ | 120–40 /0,1 (trans) |
| 22 | 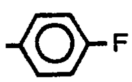 | $CH_3$ | H | H | H | $-(CH_2)_4-$ | 120–30 /0,1 (trans) |
| 23 | 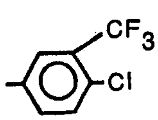 | $CH_3$ | H | H | H | $-(CH_2)_4-$ | 130 /0,1 (trans) |
| 24 | 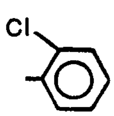 | $CH_3$ | H | H | H | $-(CH_2)_2-$ | 120 /0,1 (cis) |
| 25 | 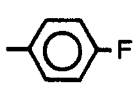 | $CH_3$ | H | H | H | $-(CH_2)_2-$ | 110 /0,1 (cis) |

TABELLE 1 (Fortsetzung)

| Beispiel Nr. | $R^1$ | $R^2$ | X | $Y^1$ | $Y^2$ | A | Kp(°C)/mm Hg–Säule |
|---|---|---|---|---|---|---|---|
| 26 | 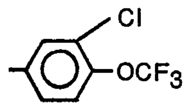 | $CH_3$ | H | H | H | $-(CH_2)_4-$ | 120 /0,1 (trans) |
| 27 | 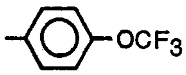 | $CH_3$ | H | H | H | $-(CH_2)_4-$ | 130–50 /0,1 (trans) |
| 28 | 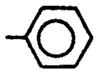 | $CH_3$ | H | H | H | $-(CH_2)_2-$ | 80 /0,1 (cis) |
| 29 | 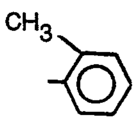 | $CH_3$ | H | H | H | $-(CH_2)_2-$ | 110 /0,1 (cis) |
| 30 | 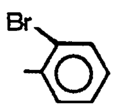 | $CH_3$ | H | H | H | $-(CH_2)_2-$ | 130 /0,1 (cis) |
| 31 | 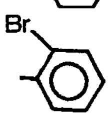 | $-CH_2-$ 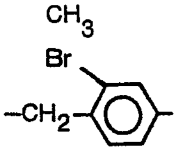 | H | H | H | $-(CH_2)_2-$ | 180 /0,1 (cis) |
| 32 | 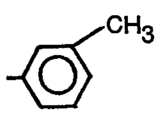 | $CH_3$ | H | H | H | $-(CH_2)_2-$ | 120 /0,1 (cis) |
| 33 | 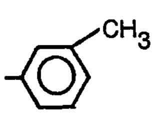 | $-CH_2-$ 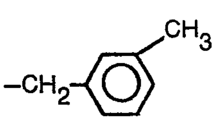 | H | H | H | $-(CH_2)_2-$ | 160 /0,1 (cis) |
| 34 | 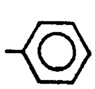 | $CH_3$ | H | H | H | $-(CH_2)_4-$ | 92 /0,1 (cis /trans) |
| 35 | 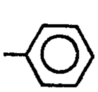 | $-CH_2-$ 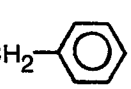 | H | H | H | $-(CH_2)_4-$ | 152–57 /0,1 (cis /trans) |
| 36 | 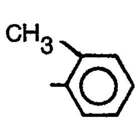 | $CH_3$ | H | H | H | $-(CH_2)_4-$ | 105–10 /0,1 (cis /trans) |

TABELLE 1 (Fortsetzung)

| Beispiel Nr. | $R^1$ | $R^2$ | X | $Y^1$ | $Y^2$ | A | Kp(°C)/mm Hg–Säule |
|---|---|---|---|---|---|---|---|
| 37 | 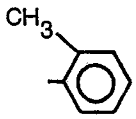 | 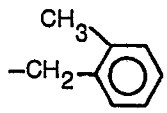 | H | H | H | $-(CH_2)_4-$ | 156–60/0,1 (cis/trans) |
| 38 | 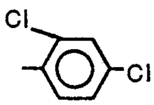 | $CH_3$ | H | H | H | $-(CH_2)_4-$ | 150/0,1 (cis) |
| 39 | 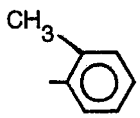 | $CH_3$ | H | H | H | $-(CH_2)_4-$ | 100/0,1 (cis) |
| 40 | 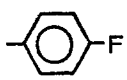 | $CH_3$ | H | H | H | $-(CH_2)_4-$ | 115/0,2 (cis) |
| 41 | 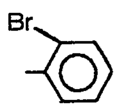 | $CH_3$ | H | H | H | $-(CH_2)_4-$ | 170/0,1 (cis) |
| 42 | 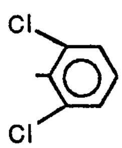 | $CH_3$ | H | H | H | $-(CH_2)_4-$ | 135/0,1 (cis) |
| 43 | 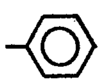 | 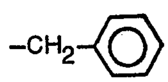 | H | H | H | $-(CH_2)_2-$ | 150/0,7 (cis) |
| 44 | 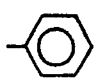 | $CH_3$ | H | H | H | 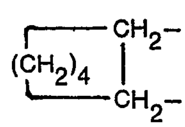 | 120/0,1 (cis) |
| 45 | 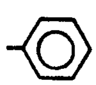 | n-$C_4H_9$ | H | H | H | $-(CH_2)_4-$ | 125/0,2 (cis) |
| 46 | 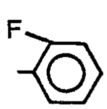 | $C_2H_5$ | H | H | H | $-(CH_2)_4-$ | 120/0,2 (cis) |

TABELLE 1 (Fortsetzung)

| Beispiel Nr. | $R^1$ | $R^2$ | X | $Y^1$ | $Y^2$ | A | Kp(°C)/mm Hg–Säule |
|---|---|---|---|---|---|---|---|
| 47 | 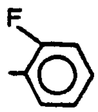 | i-$C_3H_7$ | H | H | H | $-(CH_2)_4-$ | 125/0,2 (cis) |
| 48 | 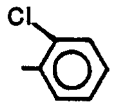 | i-$C_3H_7$ | H | H | H | $-(CH_2)_4-$ | 130/0,2 (cis) |
| 49 | 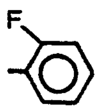 | $-CH_2-CH=CH_2$ | H | H | H | $-(CH_2)_4-$ | 125/0,2 (cis) |
| 50 | 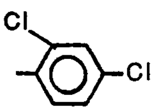 | i-$C_3H_7$ | H | H | H | $-(CH_2)_4-$ | 150/0,2 (cis) |
| 51 | 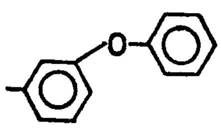 | $CH_3$ | H | H | H | $-(CH_2)_4-$ | 190/0,2 (cis) |
| 52 | 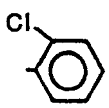 | $CH_3$ | H | H | H | $-(CH_2)_4-$ | 125/0,2 (cis) |
| 53 | 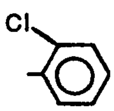 | $CH_3$ | H | H | H | $-(CH_2)_5-$ | 134/0,15 (trans) |
| 54 | 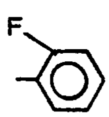 | $CH_3$ | H | H | H | $-(CH_2)_5-$ | 112/0,2 (trans) |
| 55 | 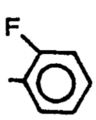 | $-CH_2-$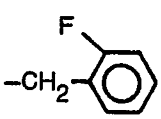 | H | H | H | $-(CH_2)_3-$ | 153/0,1 (cis) |
| 56 | 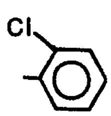 | $-CH_2-$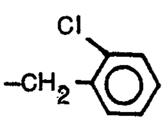 | H | H | H | $-(CH_2)_3-$ | 185/0,2 (cis) |

TABELLE 1 (Fortsetzung)

| Beispiel Nr. | $R^1$ | $R^2$ | X | $Y^1$ | $Y^2$ | A | Kp(°C)/mm Hg–Säule |
|---|---|---|---|---|---|---|---|
| 57 | 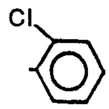 | $-CH_2-$ 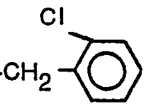 | H | H | H | $-(CH_2)_3-$ | Oel (cis / trans) |
| 58 | 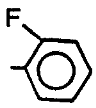 | $C_2H_5$ | H | H | H | $-(CH_2)_3-$ | 94 / 0,1 (cis) |
| 59 | 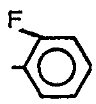 | $-CH_2-$ 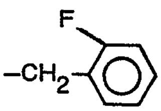 | H | H | H | $-(CH_2)_5-$ | 159 .0,1 (cis) |
| 60 | 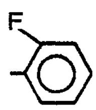 | $-CH_2-$ 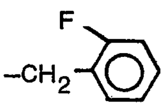 | H | H | $C_2H_5$ | $-(CH_2)_3-$ | 145 / 0,1 (cis) |
| 61 | 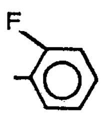 | $CH_3$ | H | H | H | $-(CH_2)_5-$ | 99 / 0,1 (cis) |
| 62* | 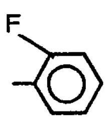 | $CH_3$ | H | H ($C_2H_5$) | $C_2H_5$ (H) | $-(CH_2)_3-$ | 109 / 0,1 (cis)* |
| 63 | 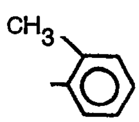 | $CH_3$ | H | H | H | $-(CH_2)_3-$ | 102 / 0,1 (cis) |
| 64 | 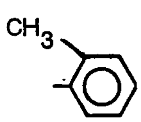 | $CH_3$ | H | H | H | $-(CH_2)_5-$ | 115–30/0,06 (cis) |
| 65 | 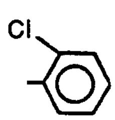 | $CH_3$ | H | H | H | $-(CH_2)_5-$ | 135–48 / 0,1 (cis) |
| 66 | 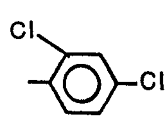 | $CH_3$ | H | H | H | $-(CH_2)_5-$ | 136–47 / 0,05 (cis/trans) |

TABELLE 1 (Fortsetzung)

| Beispiel Nr. | $R^1$ | $R^2$ | X | $Y^1$ | $Y^2$ | A | Kp(°C)/mm Hg–Säule |
|---|---|---|---|---|---|---|---|
| 67 | 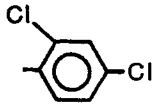 | $CH_3$ | H | H | H | $-(CH_2)_3-$ | 129 /0,3<br>(cis) |
| 68 | 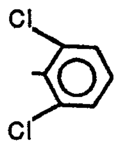 | 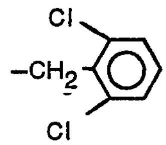 | H | H | H | $-(CH_2)_3-$ | 205–15 /0,1<br>(cis) |
| 69 | 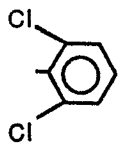 | $CH_3$ | H | H | H | $-(CH_2)_3-$ | 129 /0,1<br>(cis) |
| 70 | 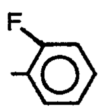 | 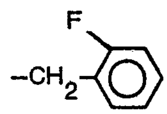 | H | H | $C_2H_5$ | $-(CH_2)_5-$ | 160 /0,1<br>(cis) |
| 71* | 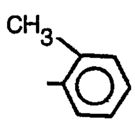 | $CH_3$ | H | H<br>($CH_3$) | $CH_3$<br>(H) | $-(CH_2)_4-$ | 114 /0,1<br>(cis)* |
| 72* | 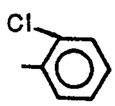 | $CH_3$ | H | H<br>($CH_3$) | $CH_3$<br>(H) | $-(CH_2)_4-$ | 127/0,2<br>(cis)* |
| 73* | 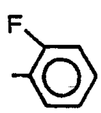 | $CH_3$ | H | H<br>($C_2H_5$) | $C_2H_5$<br>(H) | $-(CH_2)_4-$ | 122 /0,2<br>(cis)* |
| 74* | 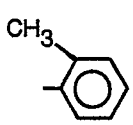 | $CH_3$ | H | H<br>($C_2H_5$) | $C_2H_5$<br>(H) | $-(CH_2)_4-$ | 127/0,1<br>(cis)* |
| 75 | 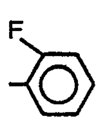 | 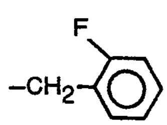 | H | H | $C_2H_5$ | $-(CH_2)_4-$ | 166 /0,1<br>(cis) |
| 76 | 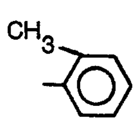 | 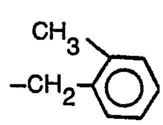 | H | H | $C_2H_5$ | $-(CH_2)_4-$ | 168 /0,1<br>(cis) |

TABELLE 1 (Fortsetzung)

| Beispiel Nr. | $R^1$ | $R^2$ | X | $Y^1$ | $Y^2$ | A | Kp(°C)/mm Hg–Säule |
|---|---|---|---|---|---|---|---|
| 77 | 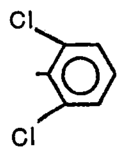 | $-CH_2$ 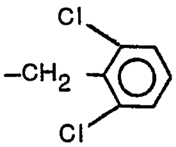 | H | H | $C_2H_5$ | $-(CH_2)_4-$ | 220–25 /0,1 (cis) |
| 78* | 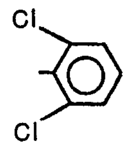 | $CH_3$ | H | H ($C_2H_5$) | $C_2H_5$ (H) | $-(CH_2)_4-$ | 152 /0,1 (cis)* |
| 79 | 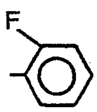 | $-CH_2$ 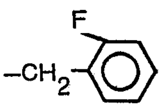 | H | H | 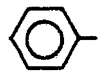 | $-(CH_2)_4-$ | 210 /0,1 (cis) |
| 80* | 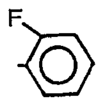 | $CH_3$ | H | H 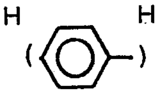 | 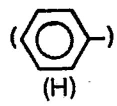 (H) | $-(CH_2)_4-$ | 144 /0,1 (cis) * |
| 81* | 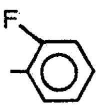 | $C_2H_5$ | H | H 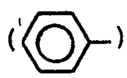 | 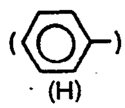 (H) | $-(CH_2)_4-$ | 152 /0,1 (cis)* |
| 82 | 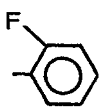 | $-CH_2$ 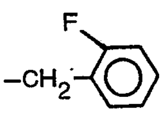 | H | H | H | $-CH_2-CH=CH-CH_2-$ | 140 /0,2 (cis) |
| 83 | 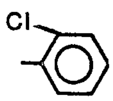 | $CH_3$ | H | H | H | $-CH_2-CH=CH-CH_2-$ | 145 /0,3 (cis) |
| 84 | 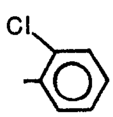 | $-CH_2$ 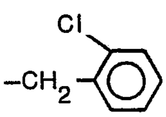 | H | H | H | $-CH_2-CH=CH-CH_2-$ | 195 /0,3 (cis) |
| 85 | 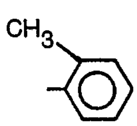 | $CH_3$ | H | H | H | $-CH_2-CH=CH-CH_2-$ | 160 /0,2 (cis) |
| 86 | 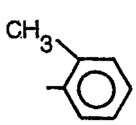 | $-CH_2$ 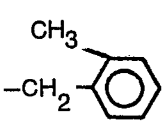 | H | H | H | $-CH_2-CH=CH-CH_2-$ | 180 /0,2 (cis) |

TABELLE (Fortsetzung)

| Beispiel Nr. | $R^1$ | $R^2$ | X | $Y^1$ | $Y^2$ | A | Kp(°C)/mm Hg–Säule |
|---|---|---|---|---|---|---|---|
| 87* | 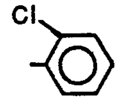 | $CH_3$ | H | H<br>(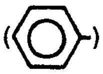) | 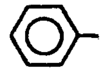<br>(H) | $-(CH_2)_4-$ | 158 /0,1<br>(cis)* |
| 88 | 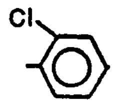 | $-CH_2-$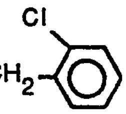 | H | H | 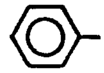 | $-(CH_2)_4-$ | 226 /0,1<br>(cis) |
| 89 | 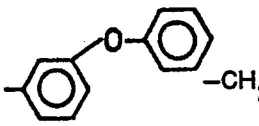 | $-CH_2-$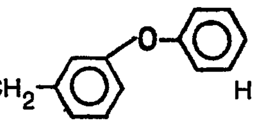 | H | H | 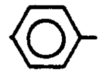 | $-(CH_2)_4-$ | 290 /0,1<br>(cis) |
| 90 | 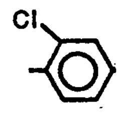 | $CH_3$ | H | H | H | $-(CH_2)_6-$ | 130 /0,1<br>(trans) |
| 91 | 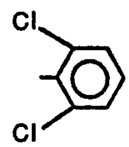 | $CH_3$ | H | H | H | $-(CH_2)_6-$ | 160 /0,1<br>(trans) |
| 92* | 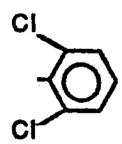 | $CH_3$ | H | H<br>(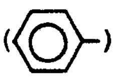) | 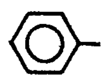<br>(H) | $-(CH_2)_4-$ | 200 /0,1<br>(cis)* |
| 93* | 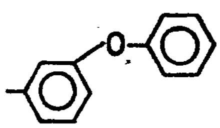 | $CH_3$ | H | H<br>(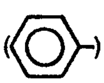) | 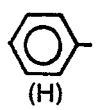<br>(H) | $-(CH_2)_4-$ | 250 /0,1<br>(cis)* |
| 94 | 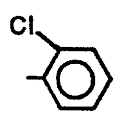 | $CH_3$ | H | H | H | $-(CH_2)_6-$ | 122 /0,1<br>(cis) |
| 95 | 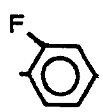 | $CH_3$ | H | H | H | $-(CH_2)_6-$ | 111 /0,1<br>(cis) |
| 96 | 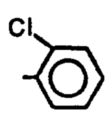 | $CH_3$ | H | H | H | $-(CH_2)_5-$ | 118 /0,09<br>(cis) |

TABELLE 1 (Fortsetzung)

| Beispiel Nr. | $R^1$ | $R^2$ | X | $Y^1$ | $Y^2$ | A | Kp(°C)/mm Hg–Säule |
|---|---|---|---|---|---|---|---|
| 97 | 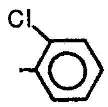 | $CH_3$ | H | H | H | $-(CH_2)_3-$ | 115 /0,13 (cis) |
| 98 | 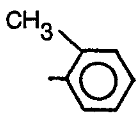 | $CH_3$ | H | H | H | $-CH_2-(CHBr)_2-CH_2-$ | 195 /0,2 (cis) |
| 99* | 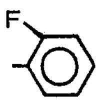 | $CH_3$ | H (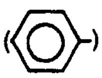) | H | 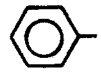 (H) | $-(CH_2)_2-$ | $n_D^{25}$ = 1,5465 (cis)* |
| 100 | 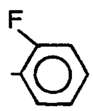 | $-CH_2-C \equiv CH$ | H | H | H | $-(CH_2)_4-$ | 101 /0,2 (cis /trans) |
| 101 | 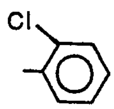 | $C_2H_5$ | H | H | H | $-CH_2-CH=CH-CH_2-$ | 101 /0,2 (cis /trans) |
| 102 | 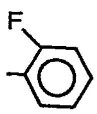 | $-CH_2-$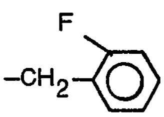 | H | H | H | $-(CH_2)_2-$ | 165 /0,2 (cis /trans) |
| 103 | 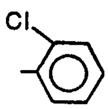 | $-CH_2-$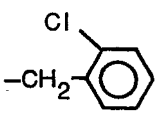 | H | H | H | $-(CH_2)_2-$ | 175 /0,2 (cis /trans) |
| 104 | 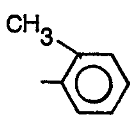 | $-CH_2-$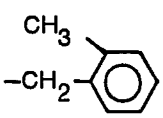 | H | H | H | $-(CH_2)_2-$ | 180 /0,2 (cis /trans) |
| 105 | 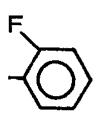 | $-CH_2-$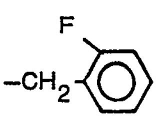 | H | H | H | $-(CH_2)_4-$ | 170 /0,2 (cis /trans) |
| 106 | 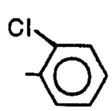 | $-CH_2-$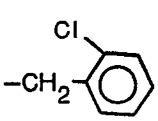 | H | H | H | $-(CH_2)_4-$ | 190 /0,3 (cis /trans) |

* Es handelt sich um Gemische von Stellungsisomeren (Stellungsisomere hinsichtlich $Y^1$ und $Y^2$)

TABELLE 1 (Fortsetzung)

| Beispiel Nr. | $R^1$ | $R^2$ | X | $Y^1$ | $Y^2$ | A | Kp(°C)/mm Hg–Säule |
|---|---|---|---|---|---|---|---|
| 107 | 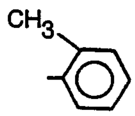 | $-CH_2-$ 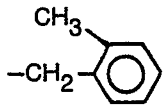 | H | H | H | $-(CH_2)_4-$ | 195 /0,2 (cis) |
| 108 | 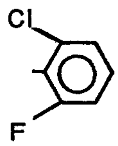 | $-CH_2-$ 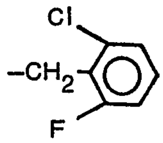 | H | H | H | $-(CH_2)_3-$ | 161 /0,1 (cis) |
| 109 | 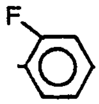 | $-(CH_2)_3-CH_3$ | H | H | H | $-(CH_2)_3-$ | 106 /0,2 (cis) |
| 110 | 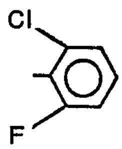 | $-CH_3$ | H | H | H | $-(CH_2)_3-$ | 103 /0,2 (cis) |
| 111 | 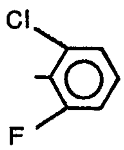 | $C_2H_5$ | H | H | H | $-(CH_2)_3-$ | 108 /0,2 (cis) |
| 112 | 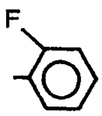 | $-CH_2-CH=CH_2$ | H | H | H | $-(CH_2)_3-$ | 99 /0,1 (cis) |
| 112 | 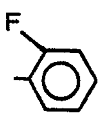 | $-(CH_2)_2-CH_3$ | H | H | H | $-(CH_2)_3-$ | 101 /0,2 (cis) |
| 114 | 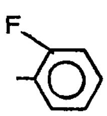 | $-CH_2-C\equiv CH$ | H | H | H | $-(CH_2)_3-$ | 110 /0,1 (cis) |
| 115 | 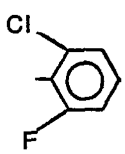 | $CH_3$ | H | H | H | $-(CH_2)_5-$ | 102 /0,06 (cis) |
| 116 | 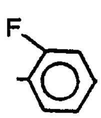 | $C_2H_5$ | H | H | H | $-(CH_2)_5-$ | 108 /0,01 (cis) |

TABELLE 1 (Fortsetzung)

| Beispiel Nr. | $R^1$ | $R^2$ | X | $Y^1$ | $Y^2$ | A | Kp(°C)/mm Hg–Säule |
|---|---|---|---|---|---|---|---|
| 117 | 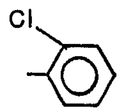 | $CH_3$ | H | H | H | 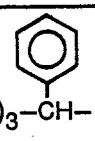 $-(CH_2)_3-CH-$ | 156–62 /0,1 (cis) |
| 118 | 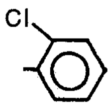 | $CH_3$ | H | H | H | $-(CH_2)_3-CH-$ \| $(CH_2)_2-CH(CH_3)_2$ | 138 /0,1 (cis) |
| 119 | 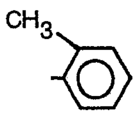 | $CH_3$ | H | H | H | 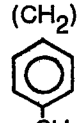 $-(CH_2)_3-CH-$ | 144 /0,1 (cis) |
| 120 | 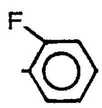 | $CH_3$ | H | H | H | 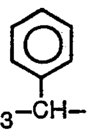 $-(CH_2)_3-CH-$ | 139–43 /0,09 (cis) |
| 121 | 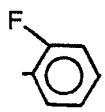 | $n-C_3H_7$ | H | H | H | $-(CH_2)_5-$ | 111 /0,1 (cis) |
| 122 | 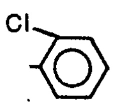 | $n-C_3H_7$ | H | H | H | $-(CH_2)_5-$ | 129 /0,1 (cis) |
| 123 | 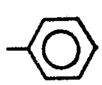 | $CH_3$ | H | H | H | $-(CH_2)_5-$ | 92 /0,1 (cis) |
| 124 | 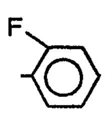 | $n-C_6H_{13}$ | H | H | H | $-(CH_2)_3-$ | 124 /0,1 (cis) |
| 125 | 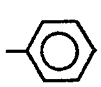 | $-CH_2-$ 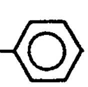 | H | H | H | $-(CH_2)_3-$ | 137 /0,1 (cis) |
| 126 | 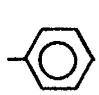 | $-CH_2-$ 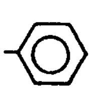 | H | H | H | $-(CH_2)_5-$ | 150 /0,1 (cis) |

TABELLE 1 (Fortsetzung)

| Beispiel Nr. | $R^1$ | $R^2$ | X | $Y^1$ | $Y^2$ | A | Kp(°C)/mm Hg-Säule |
|---|---|---|---|---|---|---|---|
| 127 | 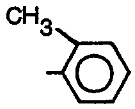 | $-CH_2-$ 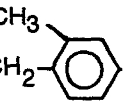 | H | H | H | $-(CH_2)_3-$ | 155 /0,1 (cis) |
| 128 | 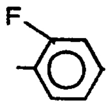 | $n-C_7H_{15}$ | H | H | H | $-(CH_2)_3-$ | 140 /0,1 (cis) |
| 129 | 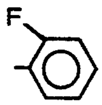 | $n-C_8H_{17}$ | H | H | H | $-(CH_2)_3-$ | 136 /0,07 (cis) |
| 130 | 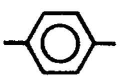 $OCH_3$ | $CH_3$ | H | H | H | $-(CH_2)_3-$ | 121 /0,2 (cis) |
| 131 | 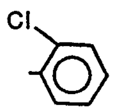 | $n-C_3H_7$ | H | H | H | $-(CH_2)_3-$ | 117 /0,1 (cis) |
| 132 | 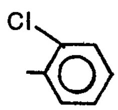 | $-CH_2-CH=CH_2$ | H | H | H | $-(CH_2)_3-$ | 122 /0,1 (cis) |
| 133 | 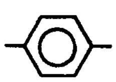 $OCH_3$ | $CH_3$ | H | H | H | $-(CH_2)_5-$ | 132 /0,09 (cis) |
| 134 | 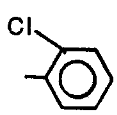 | $C_2H_5$ | H | H | H | $-(CH_2)_3-$ | 112 /0,25 (cis) |
| 135 | 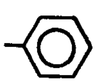 | $-CH_2-$ 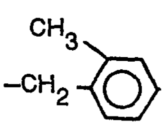 | H | H | H | $-(CH_2)_4-$ | 155–60 /0,2 (cis) |
| 136 | 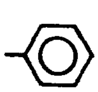 | $-CH_2-$ 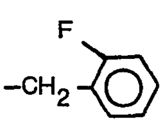 | H | H | H | $-(CH_2)_4-$ | 165–70 /0,2 (cis) |

TABELLE 1 (Fortsetzung)

| Beispiel Nr. | $R^1$ | $R^2$ | X | $Y^1$ | $Y^2$ | A | Kp(°C)/mm Hg-Säule |
|---|---|---|---|---|---|---|---|
| 137 | 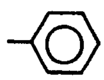 | $-CH_2-$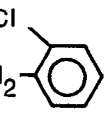 | H | H | H | $-(CH_2)_4-$ | 175/0,2 (cis) |

**Patentansprüche**

1. Benzyläther von cyclischen 1,2-Diolen der Formel

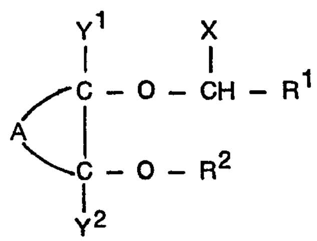

(I),

in welcher

$R^1$ für Phenyl steht, welches durch einen oder mehrere der folgenden Reste substituierte sein kann: Halogen; Alkyl, Alkoxy und Alkylthio mit jeweils 1 bis 4 Kohlenstoffatomen; Halogenalkyl, Halogenalkoxy und Halogenalkylthio mit jeweils bis zu 4 Kohlenstoffatomen und bis zu 5 Halogenatomen; durch (weiteres) Phenyl, Phenoxy oder Phenoxycarbonyl, welche jeweils durch Halogen, Alkyl oder Alkoxy mit jeweils 1 bis 2 Kohlenstoffatomen sowie durch Halogenalkyl mit bis 2 Kohlenstoff- und bis zu 3 gleichen oder verschiedenen Halogenatomen substituiert sein können; durch Alkoxycarbonyl mit 1 bis 4 Kohlenstoffatomen im Alkylteil; Alkylamino und Dialkylamino mit 1 bis 4 Kohlenstoffatomen in jedem Alkylrest; durch die Methylen-dioxy-Gruppe sowie durch den Tri-, Tetra- oder Pentamethylen-Rest;

$R^2$ für geradkettiges oder verzweigtes Alkyl mit 1 bis 8 Kohlenstoffatomen; Alkenyl und Alkinyl mit jeweils 2 bis 4 Kohlenstoffatomen; Halogenalkyl mit bis zu 2 Kohlenstoff- und bis zu 3 gleichen oder verschiedenen Halogenatomen sowie für die Gruppierung —CHX—$R^1$ steht;

X für Wasserstoff; geradkettiges oder verzweigtes Alkyl mit 1 bis 4 Kohlenstoffatomen; Alkenyl und Alkinyl mit jeweils 2 bis 4 Kohlenstoffatomen; Halogenalkyl mit bis zu 2 Kohlenstoff- und bis zu 3 gleichen oder verschiedenen Halogenatomen sowie für Phenyl, welches durch Halogen, Alkyl oder Alkoxy mit jeweils 1 oder 2 Kohlenstoffatomen sowie Halogenalkyl mit bis zu 2 Kohlenstoff- und bis zu drei gleichen oder verschiedenen Halogenatomen substituiert sein kann, steht;

$Y_1$ und $Y_2$ gleich oder verschieden sind und für Wasserstoff; geradkettiges oder verzweigtes Alkyl mit 1 bis 6 Kohlenstoffatomen sowie für Phenyl steht, welches durch die folgenden Reste substituiert sein kann; Halogen; Alkyl und Alkoxy mit jeweils 1 bis 4 Kohlenstoffatomen; Halogenalkyl, Halogenalkoxy oder Halogenalkylthio mit jeweils bis zu 4 Kohlenstoffatomen und bis zu 5 Halogenatomen; und

A für eine zwei- bis sechsgliedrige gesättigte oder ungesättigte Alkylen-Brücke steht, die durch einen oder mehrere der folgenden Reste substituiert sein kann: durch Halogen; geradkettiges oder verzweigtes Alkyl mit 1 bis 6 Kohlenstoffatomen; durch eine Di-, Tri-, Tetra- oder Pentamethylenbrücke oder Phenyl oder durch Halogen, Alkyl und Alkoxy mit jeweils 1 bis 4 Kohlenstoffatomen oder durch Halogenalkyl, Halogenalkoxy und Halogenalkylthio mit jeweils bis zu 4 Kohlenstoffatomen und bis zu 5 Halogenatomen substituiertes Phenyl.

2. Benzyläther gemäß Anspruch 1 der Formel

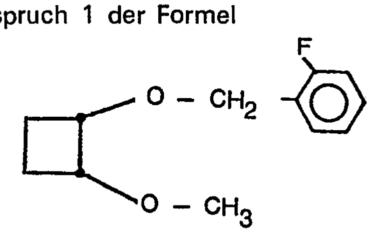

3. Benzyläther gemäß Anspruch 1 der Formel

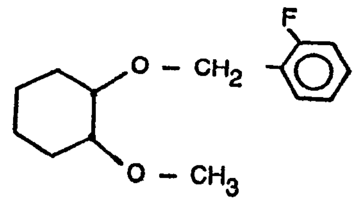

4. Benzyläther gemäß Anspruch 1 der Formel

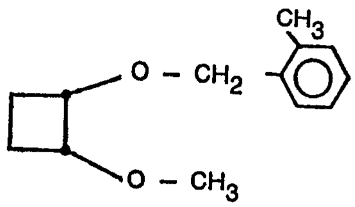

5. Benzyläther gemäß Anspruch 1 der Formel

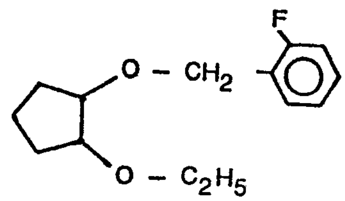

6. Verfahren zur Herstellung von Benzyläthern cyclischer 1,2-Diole der Formel (I), dadurch gekennzeichnet, daß man (A) cyclische 1,2-Diole der Formel

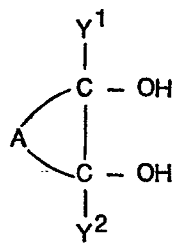

(II)

in welcher

A, $Y^1$ und $Y^2$ die oben angegebene Bedeutung haben,

mit Verbindungen der Formel

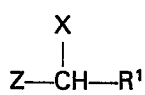

(III)

in welcher

$R^1$ und X die oben angegebene Bedeutung haben und

Z für Halogen, den Mesylat- oder Tosylat-Rest steht,

in Gegenwart einer starken Base und in Gegenwart eines Verdünnungsmittels umsetzt und gegebenenfalls die entstehenden Benzyläther der Formel

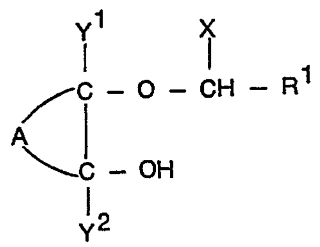

(IV)

in welcher

A, $R^1$, X

$Y^1$ und $Y^2$ die oben angegebene Bedeutung haben,
mit Verbindungen der Formel

$$Z—R^3 \quad (V)$$

in welcher

Z die oben angeführte Bedeutung hat und
$R^3$ für geradkettiges oder verzweigtes Alkyl mit 1 bis 8 Kohlenstoffatomen, Alkenyl oder Alkinyl mit jeweils 2 bis 4 Kohlenstoffatomen, Halogenalkyl mit bis zu 2 Kohlenstoff- und bis zu 3 gleichen oder verschiedenen Halogenatomen oder für die Gruppierung —CHX—$R^1$ steht, wobei $R^1$ und X die oben angegebene Bedeutung haben,

in Gegenwart einer starken Base und in Gegenwart eines Verdünnungsmittels umsetzt, oder daß man (B) cyclische 1,2-Diole der Formel (II) zuerst mit Verbindungen der Formel (V) umsetzt und die entstehenden Äther der formel

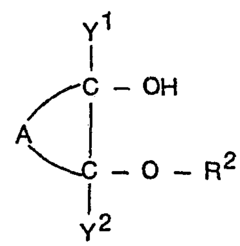
(VI)

in welcher

A, $R^2$, $Y^1$ und $Y^2$ die oben angegebene Bedeutung haben,

anschließend mit Verbindungen der Formel (III) umsetzt, jeweils unter den bei Verfahrensweg (A) angegebenen Bedingungen.

7. Herbizide Mittel gekennzeichnet durch einen Gehalt an Benzyläthern cyclischer 1,2-Diole gemäß Anspruch 1.

8. Verwendung von Benzyläthern cyclischer 1,2-Diole gemäß Anspruch 1 zur Bekämpfung unerwünschten Pflanzenwachstums.

## Claims

1. Benzyl ethers of cyclic 1,2-diols of the formula

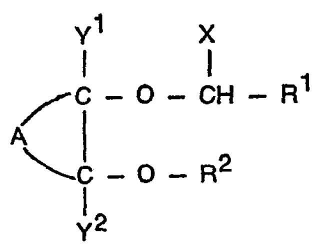
(I),

in which

$R^1$ represents phenyl which can be substituted by one or more of the following radicals: halogen (in particular fluorine, chlorine or bromine); alkyl, alkoxy and alkylthio with in each case 1 to 4 carbon atoms; halogenoalkyl, halogenoalkoxy and halogenoalkylthio with in each case up to 4 carbon atoms and up to 5 halogen atoms (in particular with up to 2 carbon atoms and up to 3 identical or different halogen atoms, halogens being in particular fluorine, chlorine and bromine); by (further) phenyl, phenoxy or phenoxycarbonyl, each of which can be substituted by halogen (in particular fluorine, chlorine or bromine), alkyl or alkoxy with in each case 1 to 2 carbon atoms and by halogenoalkyl with up to 2 carbon atoms and up to 3 identical or different halogen atoms (such as in particular fluorine and chlorine); by alkoxycarbonyl with 1 to 4 carbon atoms in the alkyl part; alkylamino and dialkylamino with 1 to 4 carbon atoms in each alkyl radical; by the methylenedioxy group and by the trimethylene, tetramethylene or pentamethylene radical.

$R^2$ represents straight-chain or branched alkyl with 1 to 8 carbon atoms; alkenyl and alkinyl with in each case 2 to 4 carbon atoms; halogenoalkyl with up to 2 carbon atoms and up to 3 identical or different halogen atoms (such as in particular fluorine and chlorine); and the grouping —CHX—$R^1$;

X represents hydrogen; straight-chain or branched alkyl with 1 to 4 carbon atoms; alkenyl and

alkinyl with in each case 2 to 4 carbon atoms; halogenoalkyl with up to 2 carbon atoms and up to 3 identical or different halogen atoms (such as in particular fluorine and chlorine); and phenyl, which can be substituted by halogen, alkyl or alkoxy with in each case 1 or 2 carbon atoms, and halogenoalkyl with up to 2 carbon atoms and up to 3 identical or different halogen atoms (such as in particular fluorine and chlorine);

$Y^1$ and $Y^2$ are identical or different and represent hydrogen; straight-chain or branched alkyl with 1 to 6 carbon atoms and phenyl which can be substituted by the following radicals: halogen; alkyl and alkoxy with in each case 1 to 4 carbon atoms; halogenoalkyl, halogenoalkoxy or halogenoalkylthio with in each case up to 4 carbon atoms and up to 5 halogen atoms (preferably with up to 2 carbon atoms and up to 3 identical or different halogen atoms, in particular fluorine, chlorine and bromine); and

A represents a two- to six-membered saturated or unsaturated alkylene bridge which can be substituted by one or more of the following radicals: by halogen (in particular chlorine or bromine); straight-chain or branched alkyl with 1 to 6 carbon atoms; by a dimethylene, trimethylene, tetramethylene or pentamethylene bridge or phenyl or by phenyl which is substituted by halogen, alkyl and alkoxy with in each case 1 to 4 carbon atoms, or by halogenoalkyl, halogenoalkoxy and halogenoalkylthio with in each case up to 4 carbon atoms and up to 5 halogen atoms (in particular with up to 2 carbon atoms and up to 3 identical or different halogen atoms, halogens being, in particular, fluorine, chlorine and bromine).

2. Benzyl ethers according to claim 1 of the formula

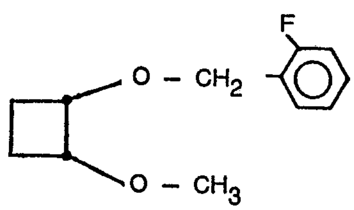

3. Benzyl ethers according to claim 1 of the formula

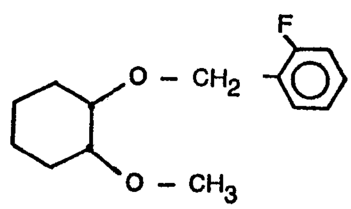

4. Benzyl ethers according to claim 1 of the formula

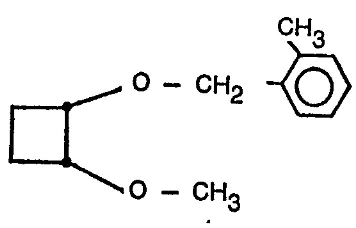

5. Benzyl ethers according to claim 1 of the formula

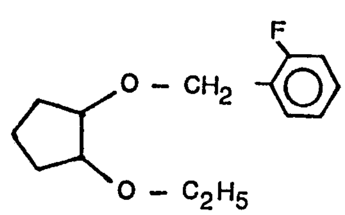

6. Process for the preparation of benzyl ethers of cyclic 1,2-diols of the formula (I), characterised in that

(A) cyclic 1,2-diols of the formula

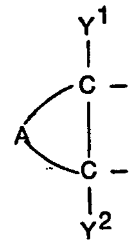

(II)

in which

A, $Y^1$ and $Y^2$ have the meaning indicated above, are reacted with compounds of the formula

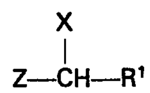

(III)

in which

$R^1$ and X have the meaning indicated above and

Z represents halogen (in particular chlorine, bromine or iodine) or the mesylate or tosylate radical, in the presence of a strong base and in the presence of a diluent and the benzyl ethers formed of the formula

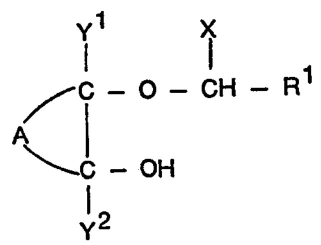

(IV)

in which

A, $R^1$, X

$Y^1$ and $Y^2$ have the meaning indicated above,

are optionally reacted with the compounds of the formula

$$Z—R^3 \quad (V)$$

in which

Z has the meaning indicated above and

$R^3$ represents straight-chain or branched alkyl with 1 to 8 carbon atoms, alkenyl or alkinyl with in each case 2 to 4 carbon atoms, halogenoalkyl with up to 2 carbon atoms and up to 3 identical or different halogen atoms (such as in particular fluorine and chlorine) or the grouping —CHX—$R^1$,

$R^1$ and X having the meaning indicated above,

in the presence of a strong base and in the presence of a diluent, or in that

B) cyclic 1,2-diols of the formula (II) are first reacted with compounds of the formula (V) and the resulting ethers of the formula

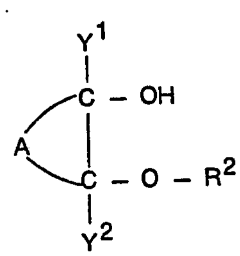

(VI)

in which

A, $R^2$, $Y^1$ and $Y^2$ have the meaning indicated above, are then reacted with compounds of the formula (III), in each case under the conditions indicated above for process route (A).

7. Herbicidal composition characterised in that it contains benzyl ethers of cyclic 1,2-diols according to claim 1.

8. Use of benzyl ethers of cyclic 1,2-diols according to claim 1 for combating undesired plant growth.

**Revendications**

1. Ethers benzyliques de 1,2-diols cycliques de formule

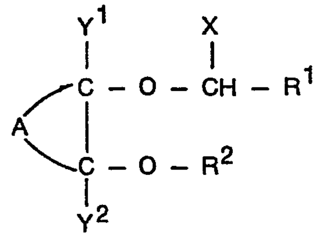

(I),

dans laquelle $R^1$ représente un reste phényle qui peut être substitué par un ou plusieurs des restes suivants:

halogène (en particulier fluor, chlore ou brome); alkyle, alcoxy et alkylthio chacun en $C_1$—$C_4$; halogénoalkyle, halogénoalcoxy et halogénoalkylthio chacun jusqu'en $C_4$ et contenant jusqu'à 5 atomes d'halogène (en particulier jusqu'en $C_2$ et contenant jusqu'à 3 atomes d'halogène identiques ou différents, en particulier fluor, chlore et brome); par un (autre) reste phényle, phénoxy ou phénoxycarbonyle, qui peut chaque fois être substitué par un halogène (en particulier fluor, chlore ou brome), un reste alkyle ou alcoxy, chacun en $C_1$ ou $C_2$, ainsi qu'un reste halogénoalkyle jusqu'en $C_2$ et contenant jusqu'à 3 atomes d'halogène identiques ou différents (en particulier fluor et chlore); par un reste (alcoxy en $C_1$—$C_4$) carbonyle; alkylamino en $C_1$—$C_4$ et di(alkyl en $C_1$—$C_4$)amino; par le groupe méthylènedioxy ainsi que le reste tri-, tétra- ou pentaméthylène;

$R^2$ représente un reste alkyl à chaîne droite ou ramifiée en $C_1$—$C_8$; alcényle et alcynyle chacyn en $C_2$—$C_4$; halogénoalkyle jusqu'en $C_2$ et contenant jusqu'à 3 atomes d'halogène identiques ou différents (en particulier fluor et chlore) ainsi que le groupement —CHX—$R^1$;

X représente l'hydrogène; un reste alkyle à chaîne droite ou ramifiée en $C_1$—$C_4$; alcényle et alcynyle chacun en $C_2$—$C_4$; halogénoalkyle jusqu'en $C_2$ et contenant jusqu'à 3 atomes d'halogène identiques ou différents (en particulier fluor et chlore) ainsi que phényle qui peut être substitué par un halogène ou un groupe alkyle ou alcoxy chacun en $C_1$ ou $C_2$ ainsi qu'halogénoalkyle jusqu'en $C_2$ et contenant jusqu'à 3 atomes d'halogène identiques ou différents (en particulier fluor et chlore);

$Y_1$ et $Y_2$ sont identiques ou différents et représentent l'hydrogène; un reste alkyle à chaîne droite ou ramifiée en $C_1$—$C_6$ ainsi que phényle, qui peut être substitué par les restes suivants: halogène, alkyle et alcoxy chacun en $C_1$—$C_4$; halogénoalkyle, halogénoalcoxy ou halogénoalkylthio chacun jusqu'en $C_4$ et contenant jusqu'à 5 atomes d'halogène (de préférence jusqu'en $C_2$ et contenant jusqu'à 3 atomes d'halogène identiques ou différents en particulier fluor, chlore et brome); et

A représente un pont alkylène saturé ou insaturé à 2—6 chaînons, qui peut être substitué par un ou plusieurs des restes suivants: halogène (en particulier chlore ou brome); alkyle à chaîne droite ou ramifiée en $C_1$—$C_6$; un pont di-, tri-, tétra- ou pentaméthylène ou un groupe phényle ou phényle substitué par halogène, alkyle et alcoxy chacun en $C_1$—$C_4$ ou halogénoalkyle, halogénoalcoxy et halogénoalkylthio chacun jusqu'en $C_4$ et contenant jusqu'à 5 atomes d'halogène (en particulier jusqu'en $C_2$ et contenant jusqu'à 3 atomes d'halogène identiques ou différents, en particulier fluor, chlore et brome).

2. Ethers benzyliques selon la revendication 1 répondant à la formule

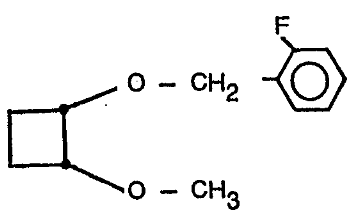

3. Ethers benzyliques selon la revendication 1 répondant à la formule

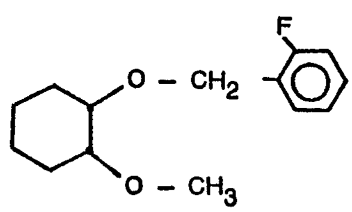

4. Ethers benzyliques selon la revendication 1 répondant à la formule

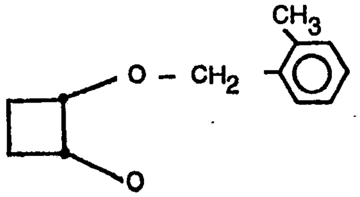

5. Ethers benzyliques selon la revendication 1 répondant à la formule

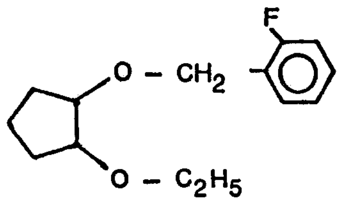

6. Procédé pour la préparation d'éthers benzyliques de 1,2-diols cycliques de formule (I), charactérisé en ce que (A) on fait réagir des 1,2-diols cycliques de formule

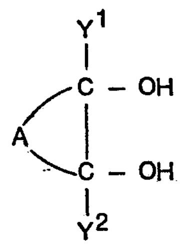

(II)

dans laquelle A, $Y^1$ et $Y^2$ sont tels que définis ci-dessus avec des composés de formule

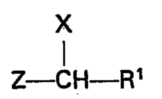

(III)

dans laquelle $R^1$ et X sont tels que définis ci-dessus et Z représente un halogène (en particulier chlore, brome ou iode), le reste mésylate ou tosylate,
en présence d'une base forte et en présence d'un agent diluant et éventuellement on fait réagir les éthers benzyliques formés de formule

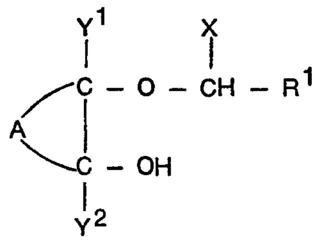

(IV,

dans laquelle A, $R^1$, X, $Y^1$ et $Y^2$ sont tels que définis ci-dessus avec des composés de formule

$$Z—R^3 \qquad (V)$$

dans laquelle Z est tel que défini ci-dessus, et
$R^3$ représente un groupe alkyle à chaîne droite ou ramifiée en $C_1$—$C_8$, alcényle ou alcynyle chacun en $C_2$—$C_4$, halogénoalkyle jusqu'en $C_2$ et contenant jusqu'à 3 atomes d'halogène identiques ou différents (en particulier fluor et chlore) ou le groupement —CHX—$R^1$, dans lequel $R^1$ ex X sont tels que définis ci-dessus,
en présence d'une base forte et en présence d'un agent diluant, ou bien en ce que

(B) on fait d'abord réagir des 1,2-diols cycliques de formule (II) avec des composés de formule (V) et on fait ensuite réagir les éthers formés de formule

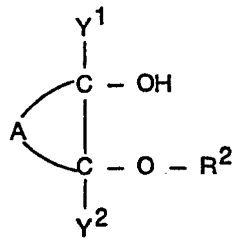

(VI)

dans laquelle A, $R^2$, $Y^1$ et $Y^2$ sont tels que définis ci-dessus avec les composés de formule (III), chaque fois dans les conditions indiquées dans le mode opératoire (A).

7. Agents herbicides caractérisés en ce qu'ils contiennent des éthers benzyliques de 1,2-diols cycliques selon la revendication 1.

8. Utilisation d'éthers benzyliques de 1,2-diols cycliques selon la revendication 1 pour la lutte contre la croissance de plantes indésirables.